# EUROPEAN PATENT APPLICATION

(11) **EP 3 563 877 A1**
(43) Date of publication of application: **06.11.2019**
(21) Application number: 19171837.8
(22) Date of filing: 30.04.2019
(51) Int. Cl.: A61L 9/12, A01M 1/20

(54) **DIFFUSER WITH CAP AND FRAMEWORK ASSEMBLY**

(30) Priority: 04.05.2018 ES 201830628 U
(71) Applicant: Zyxtudio Diseño e Innovación, S.L., 46010 Valencia (ES)
(72) Inventor: Blasco Feo, Vicente, 46010 Valencia (ES)
(74) Representative: Soler Lerma, Santiago

(57) **Abstract**

The invention, belongs to the field of diffusers. As the name indicates, relates to a cap and framework assembly of the type used in diffusers wherein a strip of impregnable material or wick is impregnated with the material inside a container. This wick is secured to a framework joined to the cap which closes the container so that when opening the container and removing the cap, the framework and wick also come out, with the framework comprising means to remain fixed at the desired height thanks to its flexibility and geometry which allows its interaction with the container's internal geometry.

## Description

The invention, as the name indicates, relates to a cap and framework assembly of the type used in diffusers wherein a strip of impregnable material or wick is impregnated with the material inside a container. This wick is secured to a framework joined to the cap which closes the container so that when opening the container and removing the cap, the framework and wick also come out, with the framework comprising means to remain fixed at the desired height thanks to its flexibility and geometry which allows its interaction with the container's internal geometry.

This invention belongs to the field of diffusers.

### BACKGROUND OF THE INVENTION

Diffusers have been known for some time that comprise a container with the substance to diffuse, a wick which is impregnated with the substance, a framework which serves as holder of the wick and which is attached to the cap so that on removing the cap the framework and wick come out of the container and may be fixed at the desired height thanks to the framework having a certain flexibility and geometry which is complemented with the internal geometry of the container mouth.

The user handles the wick from the cap so that it avoids him from getting stained and, once he no longer wants to use the diffuser, he recloses the container with the cap with the wick and framework remaining inside the container.

The following are some of the typical problems with these diffusers:
The necessary clearances to facilitate the exit and entry of the framework mean that the cap and framework assembly can wobble generating instability to the diffuser device, especially when there is little substance to diffuse and, therefore, the wick is only impregnated in its lower part and the user, to expose the impregnated part of the wick to the action of the outside air, must raise the framework, increasing the height of the assembly.

An example of some patents that give solutions of this type are US2452424 wherein the framework, which has two legs that tend to open, is made in a flexible cable which, on deforming, allows the entry and exit of the framework through the container mouth but which, in absence of vertical force, either upward or downward, is fixed by friction against the inner part of the container neck. In this case, framework and cap are not joined.

A more evolved device is that object of patent US3091396 which has a framework with laterally oriented saw-teeth so that these saw-teeth contribute to fixing the framework and the wick at the desired height.

Both in one case and in the other, the assembly's stability is compromised as there is no means that avoid the wobbling of the framework, with it being possible for the container to topple over and spill the liquid.

To avoid spills and contribute to more firmly securing the framework with the wick, avoiding the wobbling, we have patent US4742960 comprising a narrowing in the container neck so that, on the one hand, by pressure, the framework is better secured with the wick and, on the other, in the event of the container accidentally falling, the exit orifice is practically sealed with the wick and the framework so that the spill would be minimized.

More recently is Belgian patent BE1007062 which relates to a diffuser wherein the framework and the cap are joined by clipping so that it enables that, in its manufacturing, framework and wick go inside the container when it has to be filled, the cap being screwed and joined to the framework at a later time.

The existence of an object in the container mouth, such as the upper end of the framework, can generate problems for the filling, and it is for this reason that framework ends have been devised with ring-shaped form so that it facilitates the filling although, on the one hand, there are still problems of splashes, and, on the other, the clips of this type of framework, at least those known to date, are not efficient as they require a number of complex undercuts in the cap moulds or the framework which generate a high number of defective product and high material consumption.

Furthermore, neither do the current frameworks, which have laterally oriented saw-teeth, resolve the wobbling, with the assembly's stability being compromised.

### DESCRIPTION OF THE INVENTION

To resolve the stated problems, a cap and framework assembly for the wick is proposed which favours the stability of the diffuser device avoiding wobbling of the framework, and this assembly also has clipping elements that allowing saving in manufacturing costs both in moulds and in material and are more effective than those known to date, favouring the filling of the container.

Hence, the assembly comprises:
1. A cap which has, in its inner ceiling, a downward projection with a geometry complementary with the upper base of a framework so that the cap and the framework clip, remaining joined.
2. A framework comprising:
   a. An upper base which, in turn, comprises a ring wherein the geometry inside the ring is complementary with the downward projection of the ceiling inside the cap and said downward projection clips with the inner part of the ring.
   b. A series of orifices that vertically traverse the ring, allowing the outlet of air from inside the container whilst the container is filled with the substance to diffuse through the central orifice of the ring.
   c. A structure that extends vertically downwards from the ring and comprises two arms, preferably parallel to one another and which preferably start from diametrically opposite positions of the ring of the upper base, wherein these arms comprise:
      i. Saw-teeth in their two longitudinal edges, in front and rear position.
      ii. An extension in the lower end of each one of the arms, by way of a tab, with each one of them extending towards the front and rear part of the framework and wherein these tabs have a harpoon-type geometry with curvature in its lower part and a right angle in its upper part.

The device of the type described allows the framework to be inserted in the container, with the wick already suitably attached to it, before the container is filled with the fluid to diffuse.

Thanks to the form of the ring of the upper base of the framework, the container filling operation can be performed without problems through the ring's central orifice.

To avoid the formation of air bubbles that may generate filling problems, the ring of the upper part of the framework comprises, in addition to the central orifice, at least one through-cavity, understanding as such that which allows communication between the lower and upper part of the ring, allowing, during the filling operation, the exit of pre-existing air in the container.

The through-cavities may be perimetral by way of open channel that runs through the outer side of the ring or through-holes in the ring body.

Once the container has been filled, the cap is incorporated which, when completely screwed, clips with the framework as the inner geometries of the ring are complementary with that of the downward projection of the ceiling inside the cap.

It is important to highlight that the cap must be clipped without losing its possibility of rotation since, otherwise, it would prevent its screwing or unscrewing due to the fact that the framework does not rotate due to the narrowing of the container neck.

So that the cap can rotate, the clip must also act by way of bushing and, in particular in this case, a very small clip has been considered to avoid friction and save material, so that the clipping occurs in the inner part of the ring,
Thanks to this clipping, cap and framework are joined together so that if the user removes the cap he pulls the framework and the wick.

The saw-teeth which traverse the front and rear edge of each arm of the framework, adjust the front and rear part of the inside of the container neck, especially in its narrowing area, allowing that the framework can be fixed at the height desired by the user.

On adjusting the front and rear part of each one of the framework's downward arms to the container, four points of friction are generated between the framework and the container, so that wobbling of the cap and the framework are avoided, increasing the assembly's stability.

As the lower tabs have a straight upper geometry, they act as limit to avoid the complete exit of the framework, avoiding spills, however as it has a curved lower geometry, it facilitates the introduction of the framework in the container.

Of particular importance is the direction of the tabs, as if they were projected towards the sides, as occurs in other known devices, on the one hand, it would offer less resistance to upward push not being as safe when avoiding accidental removal from the framework and, on the other hand, they do not collaborate in attaching the wick, whilst with the proposed configuration of front and rear extension, these two purposes are achieved.

### BRIEF DESCRIPTION OF THE FIGURES

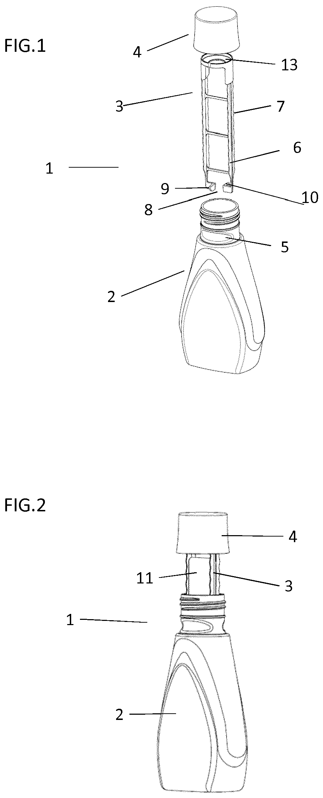

FIGURE 1 shows an exploded view of the diffuser device (1) comprising a container (2) a framework (3) and a cap (4) wherein the container has a narrowing (5) in the neck, the framework has lower arms (6) with saw-teeth (7) in its front and rear edges and has tabs (8) in its lower end wherein one tab has a front extension (9) and the another tab a rear extension (10). The wick has not been included in this figure.
FIGURE 2 shows the device (1) in operation, observing the cap (4), the framework (3), which in this case has mounted a wick (11), and the container (2).

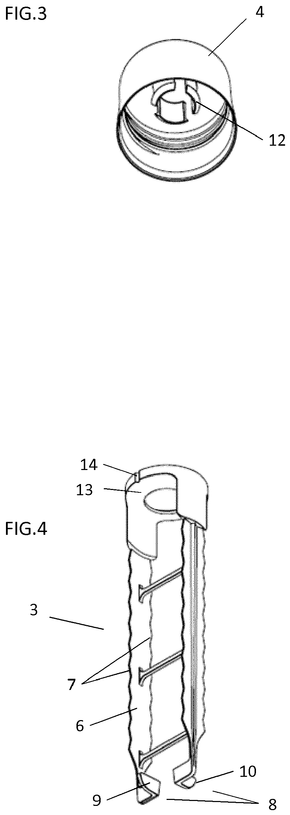

FIGURE 3 shows in detail the cap (4) wherein the downward projection (12) is observed, complementary with the inner part of the upper ring of the framework.
FIGURE 4 shows in detail the framework (3) wherein the upper ring (13) is observed, with the through-cavities (14), the lower arms (6) with saw-teeth (7) in its front and rear edges.

In the lower ends of the arms are the tabs (8) of which one tab has a front extension (9) and the other tab has a rear extension (10).

### DESCRIPTION OF AN EMBODIMENT OF THE INVENTION

An embodiment of the invention is stated below, which is not limiting but explanatory so that any other form of executing it which meets the claimed technical conditions would enter in the scope of protection.

The invention relates to an improved diffuser device with cap and framework assembly, of the type comprising a container, a cap which in use is joined to a framework, with that framework serving as a holder for a wick which is impregnated with the substance housed in the container.

So that there is a diffusion of the substance, the wick must come into contact with the air and, for said purpose, the device has a framework that holds the wick and comprises means to be able to be fixed at different heights protruding from the container mouth.

It is desirable that this framework does not wobble to avoid stability problems.

To facilitate handling the framework, it is joined to the cap so that the user at no time needs to touch the framework, only the cap.

It is typical that, within the manufacturing process, during filling the container, the framework with the wick is already inside it.

To facilitate the filling, the framework has been provided with a specific upper base that facilitates the entry of fluid since on the one hand it eliminates elements that may get in the way of the fluid path and, on the other, it allows the outlet of the pre-existing air in the container without bubbles forming that may give rise to splashes or overflows.

Hence, the device (1) comprises:
1. A container (2) which has a narrowing (5) in the neck.
2. A framework (3) which has;
   a. An upper ring (13) in which perimeter through-cavities (14) have been made, which allow the outlet of the pre-existing air in the container during the filling operations;
   b. Two lower arms (6) comprising:
   c. Saw-teeth (7) in its front and rear edge.
   d. In the lower end of each one of the arms, tabs (8) one in each arm, one of the tabs having a front extension (9) and the tab of the other arm having a rear extension (10).
3. A cap with a downward projection (12) made in flexible material with a harpoon geometry suitable for clipping in the inner part of the upper ring of the framework, reducing in this way the friction between cap and ring and reducing the quantity of material necessary for manufacturing the cap and the simplicity of its mould.
4. A wick held by the framework made in impregnable material.

A device comprising all these elements enables locating the framework and wick inside the container before its filling, without the filling operations being compromised by obstacles in the fluid path nor by the formation of air bubbles thanks to the perimeter through-cavities (14), which allow the exit of air whilst the filling fluid enters through the orifice inside the ring (13).

Once filled, the cap is installed, which has a downward projection (12) therein, made in flexible material with harpoon geometry that clips with the inner part of the ring generating a permanent joint but with the suitable clearance and geometry to allow cap rotation during the screwing and unscrewing operations.

With the cap and framework, which holds the impregnated wick, joined together in this way, the user can expose the wick to the air for which he will only have to lift the cap pulling the framework and wick and decide on the height at which he wants to fix the framework.

The framework is fixed thanks to the action of the saw-teeth (7) of the arms (6) of the framework, which as these teeth are disposed in the front and rear edges of each one of the arms, generate four points of friction with the container neck, allowing a firm positioning of the framework without wobbling.

The extensions (8) of the ends of the arms, with their front (9) and rear (10) extensions play a dual role as, on the one hand, they facilitate the joint between the framework and the wick and, on the other, they hinder the involuntary removal of the framework as it butts with the narrowing (5) of the container neck (2).

## Claims

1. DIFFUSER WITH CAP AND FRAMEWORK ASSEMBLY of the type comprising a container (2) with the substance to diffuse which has a narrowing (5) in its neck, a framework (3) which has downward arms (6) and which holds a wick (11) impregnated in said substance, a cap (4) joined to the framework **characterized in that** the framework comprises a ring (13) in its upper part which has through-cavities (14) which connect the lower and upper part of the ring, saw-teeth (7) in the front and rear edges of the arms (6) and tabs (8) in the lower end of the arms; a cap (4) comprising a downward projection (12) made in flexible material with a geometry complementary with the inner part of the ring.

2. DIFFUSER WITH CAP AND FRAMEWORK ASSEMBLY according to claim 1, **characterized in that** the framework, in the lower ends of the arms has tabs (8) wherein one tab has a front extension (9) and another tab a rear extension (10).

3. DIFFUSER WITH CAP AND FRAMEWORK ASSEMBLY according to claim 1, **characterized in that** the through-cavities (14) have the form of open channel which vertically runs through the side of the ring.

4. DIFFUSER WITH CAP AND FRAMEWORK ASSEMBLY according to claim 1, **characterized in that** the through-cavities (14) are through-holes made in the body of the ring.

5. DIFFUSER WITH CAP AND FRAMEWORK ASSEMBLY according to claim 1, **characterized in that** the saw-teeth (7) fit against the front and rear part of the inside of the container neck in its narrowing area (5).

6. DIFFUSER WITH CAP AND FRAMEWORK ASSEMBLY according to claim 1, **characterized in that** the lower geometry of the front and rear extensions of the tabs (8) is curved.
